(19) Europäisches Patentamt
European Patent Office
Office européen des brevets

(11) Veröffentlichungsnummer: **0 150 673**
**B1**

(12) # EUROPÄISCHE PATENTSCHRIFT

(45) Veröffentlichungstag der Patentschrift: **29.06.88**

(51) Int. Cl.⁴: **H 01 M 4/60**, C 07 F 17/00

(21) Anmeldenummer: **84810634.0**

(22) Anmeldetag: **17.12.84**

(54) **Festelektrolytzelle und mit Jod dotierte Metallkomplexe als Kathodenmaterial.**

(30) Priorität: **23.12.83 CH 6884/83**

(43) Veröffentlichungstag der Anmeldung: **07.08.85 Patentblatt 85/32**

(45) Bekanntmachung des Hinweises auf die Patenterteilung: **29.06.88 Patentblatt 88/26**

(84) Benannte Vertragsstaaten: **BE CH DE FR GB IT LI NL**

(56) Entgegenhaltungen:
**DE-A-2 046 354**

**J.C.S. CHEM. COMM., 1980, Seiten 954-955; L.-S. LIN et al.: "New class of electrically conductive metallomacrocycles: Iodine-doped dihydrodibenzo b,i 1,4,8,11 tetra-azacyclotetradecine complexes"**
**PATENTS ABSTRACTS OF JAPAN, Band 5, Nr. 82 (E-59) 754, 29. Mai 1981; & JP - A - 56 30 261 (MATSUSHITA DENKI SANGYO K.K.) 26-03-1981**

(73) Patentinhaber: **CIBA- GEIGY AG, Klybeckstrasse 141, CH- 4002 Basel (CH)**

(72) Erfinder: **Hunziker, Max, Dr., St. Alban Ring 230, CH- 4052 Basel (CH)**

**EP 0 150 673 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft eine Festelektrolytzelle mit festem Lithium oder Silber als Anode, einem festen Lithiumjodid oder Silberjodid als Elektrolyt und einem mit Jod dotierten Dibenzotetraaza-[14]-annulen-Metallkomplex als Kathodenmaterial sowie solche Metallkomplexe und ein Verfahren zu deren Herstellung.

Die Verwendung von organischen Chargetransfer-Komplexen als Kathodenmaterial in Festelektrolytzellen ist bekannt. Als technisch interessante Komplexe haben sich mit Jod dotierte Polymere wie zum Beispiel Polyvinylpyridin (PVP) und Polyvinylchinolin (PVQ) erwiesen. Diese Materialien können über das komplexgebundene Jod hinaus zusätzlich molekulares Jod bis zu einem Gesamtgehalt von 124 Mol $J_2$ pro Mol Monomer aufweisen (vgl. US-PS-4 340 651).

In der japanischen Patentveröffentlichung Sho 56-30 261 wird erwähnt, dass diese Materialien halbfest bis pastös sind und daher nur schwierig verarbeitet werden können. Insbesondere bereitet die Tablettierung der Materialien Schwierigkeiten. In der Regel werden Kathoden aus PVP-$J_2$ bei höherer Temperatur gegossen. Nur bei Jodgehalten bis höchstens 30 Mol $J_2$ pro Mol Vinylpyridin ist das Mischen im festen Zustand und eine Tablettierung ohne Zerfliessen des Materials möglich. Allerdings weisen diese aus PVP und $J_2$ fest gemischten Materialien hohe Anfangswiderstände auf, die erst im Verlaufe von Monaten wieder abnehmen (vgl. US-PS-4 148 975). Dies hat zur Folge, dass Festelektrolytzellen mit diesen Kathodenmaterialien sehr hohe Anfangswiderstände aufweisen und nur bei langsamer Entladung im Bereich von einigen µA (sehr grosse Lastwiderstände) eine brauchbare Spannung aufrechterhalten.

In der schon erwähnten japanischen Patentveröffentlichung Sho 56-30 261 werden als Kathodenmaterial für Lithiumzellen Chargetransfer-Komplexe von Jod bzw. Brom und Phthalocyanin-Metallkomplexen vorgeschlagen. Die Dotierung mit Jod verläuft sowohl in organischen Lösungsmitteln wie mit gasförmigem Jod sehr langsam und kann bis zu einem Monat dauern. Die aufgenommenen Jodmengen sind relativ klein, was zu einer, geringen Lebensdauer der Batterien führt, die zudem nur eine geringe Energiedichte aufweisen.

Es wurde nun gefunden, daß, sich mit Jod dotierte Dibenzotetraaza-[14]-annulen-Metallkomplexe hervorragend als Kathodenmaterial in Lithium/Jod-Festelektrolytzellen eignen. Die Komplexe sind einfach herstellbar, in kurzen Zeiten mit Jod dotierbar und sie weisen auch bei hohen Jodgehalten noch eine hohe Leitfähigkeit auf. Ausserdem werden im Vergleich zu den Phthalocyanin-Komplexen höhere Energiedichten erzielt. Ferner beobachtet man bei den Komplexen schon nach der Herstellung nur einen geringen Anfangswiderstand, der sich im Falle der durch Mischen oder Mahlen der festen Reaktionspartner hergestellten Chargetransfer-Komplexe im Verlauf kurzer Zeitspannen sogar noch vermindert, wobei diese Abnahme aber keinen nennenswerten Einfluss auf den Innenwiderstand einer Batterie hat.

Ein Gegenstand vorliegender Erfindung ist eine Festelektrolytzelle, enthaltend eine feste Anode aus Lithium oder Silber, einen festen Lithiumjodid- oder Silberjodid-Elektrolyt und einen Chargetransfer-Komplex aus einem Metallkomplex und Jod als Kathode, die dadurch gekennzeichnet ist, dass die Kathode aus einem mit Jod dotierten Komplex oder dessen Gemisch mit Jod entsprechend der Formel I besteht,

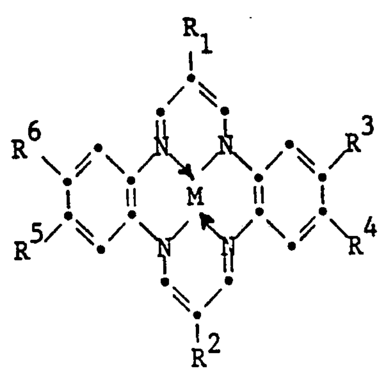 $\bullet\ x\ J_2$ (I)

worin

M ein zweiwertiges Metallatom aus der Gruppe Fe, Co, Ni, Cu, Zn, Pd und Pt ist,

$R^1$ und $R^2$ unabhängig voneinander für ein Wasserstoffatom oder unsubstituiertes oder substituiertes Alkyl, Cycloalkyl, Aryl, Aralkyl, Alkaralkyl oder Acyl stehen,

$R^3$, $R^4$, $R^5$ und $R^6$ unabhängig voneinander ein Wasserstoffatom, Alkyl, Alkoxy, Alkylthio und Alkoxycarbonyl mit 1 bis 8 C-Atomen, Aryloxy oder Halogen sind, oder $R^3$ und $R^4$ sowie $R^5$ und $R^6$ zusammen $-(CH_2)_n-$ mit n, 3, 4 oder 5, $-O-(CH_2)_m-O-$ mit m = 1 - 4, oder ein Rest der Formel -CH = CH-CH = CH- sind, und

x für eine rationale Zahle von 1 bis 300 steht.

Mit dem Komplex der Formel I wird zum Ausdruck gebracht, dass zwischen dem Metallkomplex und einem Teil des Jods ein partieller, nicht integraler Ladungstransfer stattgefunden hat und weiteres überschüssiges Jod in molekularer Form vorliegen kann.

M in Formel I ist bevorzugt Fe und besonders Cu oder Ni. Bei $^1$ und $R^2$ handelt es sich bevorzugt um gleiche Substituenten, ebenso bei $R^3$ bis $R^6$.

$R^1$ und $R^2$ als Alkyl können linear oder verzweigt sein und enthalten bevorzugt 1 bis 6, besonders 1 bis 4 C-Atome. Beispiele sind Hexyl, Pentyl, t-Butyl, sek.-Butyl, n-Butyl, n-Propyl, i-Propyl und besonders Äthyl und

Methyl.

$R^1$ und $R^2$ als Cycloalkyl enthalten bevorzugt 5 oder 6 Ringkohlenstoffatome und können z. B. Cyclopentyl oder Cyclohexyl sein.

Als Acyl enthalten $R^1$ und $R^2$ bevorzugt 2 bis 6, besonders 2 bis 4 C-Atome und können z. B. Acetyl, Propionyl oder Benzoyl sein.

Geeignete Substituenten für $R^1$ und $R^2$ sind z. B. Halogen wie Br, Cl und F, oder Alkoxy mit bevorzugt 1 bis 4 C-Atomen.

$R^1$ und $R^2$ als Aryl sind bevorzugt Phenyl, das z. B. durch Halogen, besonders Cl und F, oder Alkoxy mit 1 bis 4 C-Atomen, besonders Methoxy, substituiert sein kann. Die Substituenten befinden sich bevorzugt in para-Stellung.

$R^1$ und $R^2$ als Alkaryl sind bevorzugt Alkylphenyl, insbesondere p-Alkylphenyl mit bevorzugt 1 bis 4 C-Atomen in der Alkylgruppe. Beispiele sind p-Butylphenyl, p-Propylphenyl, p-Äthylphenyl und besonders p-Methylphenyl.

$R^1$ und $R^2$ als Alkaralkyl sind bevorzugt Alkylbenzyl mit bevorzugt 1 bis 4 C-Atomen in der Alkylgruppe, und besonders p-Alkylbenzyl, z. B. p-Methylbenzyl.

Eine bevorzugte Untergruppe der Komplexe der Formel 1 sind solche, in denen $R^3$ bis $R^6$ für ein Wasserstoffatom und $R^1$ und $R^2$ für ein Wasserstoffatom oder Alkyl, Cycloalkyl, Aryl, Aralkyl, Alkaralkyl oder Acyl stehen.

$R^3$ bis $R^6$ als Alkyl, Alkoxy, Alkylthio und Alkoxycarbonyl enthalten bevorzugt 1 bis 4 C-Atome und können linear oder verzweigt sein. Beispiele sind n-Butyl, sek.-Butyl, t-Butyl, n-Propyl, i-Propyl, Butyloxy, Propyloxy, Äthoxy, Butylthio, Propylthio, Äthylthio, Methoxycarbonyl, Äthoxycarbonyl und besonders Methyl, Äthyl, Methoxy und Methylthio. $R^3$ und $R^4$ sowie $R^5$ und $R^6$ zusammen sind bevorzugt $-(CH_2)_n-$, $-OCH_2O-$, $-OCH_2CH_2O-$ oder $-CH=CH-CH=CH-$. $R^3$ bis $R^6$ als Aryloxy sind bevorzugt Phenoxy und als Halogen bevorzugt Cl und besonders F.

In einer bevorzugten Ausführungsform stehen $R^1$ bis $R^6$ unabhängig voneinander für ein Wasserstoffatom oder eine der zuvor definierten Kohlenwasserstoffgruppen, besonders für Alkyl.

In Formel I kann x bevorzugt eine Zahl von 1 bis 200, besonders 1 bis 100 und insbesondere 2 bis 50 sein.

In einer bevorzugten Ausführungsform besteht das Kathodenmaterial aus Komplexen der Formel I, worin $R^1$ bis $R^6$ unabhängig voneinander für ein Wasserstoffatom oder Methyl und M für Cu oder Ni stehen.

Komplexe der Formel I, in denen x eine Zahl von mindestens 10 ist, sind neu. In diesen Komplexen liegt ein Überschuss an Jod vor, so daß die Chargetransfer-Komplexe in einer Matrix aus Jod verteilt sind. Diese Mischungen weisen überraschend ebenfalls eine hohe Leitfähigkeit auf und zeichnen sich durch einen niedrigen Widerstand bei der Verwendung als Kathodenmaterial in Batterien aus.

Ein weiterer Gegenstand vorliegender Erfindung ist ein mit Jod dotierter Komplex oder dessen Gemisch mit Jod entsprechend der Formel II

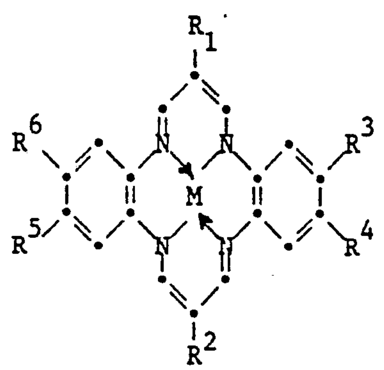 $\cdot\, y\ J_2$ (II),

worin

M ein zweiwertigen Metallatom aus der Gruppe Fe, Co, Ni, Cu, Zn, Pd und Pt ist,

$R^1$ und $R^2$ unabhängig voneinander für ein Wasserstoffatom, oder unsubstituiertes oder substituiertes Alkyl, Cycloalkyl, Aryl, Aralkyl, Alkaralkyl oder Acyl stehen,

$R^3$, $R^4$, $R^5$ und $R^6$ unabhängig voneinander ein Wasserstoffatom, Alkyl, Alkoxy, Alkylthio und Alkoxycarbonyl mit 1 bis 8 C-Atomen, Aryloxy oder Halogen sind, oder $R^3$ und $R^4$ sowie $R^5$ und $R^6$ zusammen $-(CH_2)_n-$ mit n = 3, 4 oder 5, $-O-(CH_2)_m-O-$ mit m = 1 bis 4, oder einen Rest der Formel $-CH=CH-CH=CH-$ sind, und

y für eine rationale Zahl von 10 bis 300 steht.

Für die Komplexe der Formel II gelten die gleichen Bevorzugungen wie für die Komplexe der Formel I. Die Zahl y ist bevorzugt 5 bis 200, besonders 5 bis 100 und insbesondere 5 bis 50. Die Zahl y (wie auch die Zahl x in Formel I) gibt das Molverhältnis von Metallkomplex zu molekularem Jod wieder.

Die Herstellung der mit Jod dotierten Komplexe oder deren Mischungen mit Jod entsprechend der Formeln I und II erfolgt in an sich bekannter Weise durch die Oxidation eines Metallkomplexes der Formel III

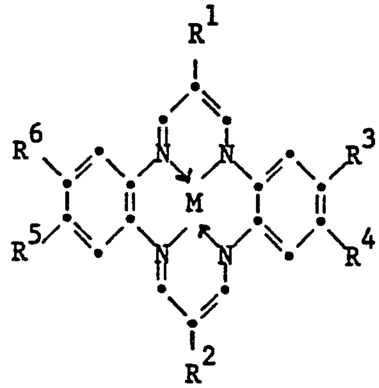

(III),

worin M und $R^1$ bis $R^6$ die zuvor angegebene Bedeutung haben, mit x bzw. y Mol molekularem Jod und gegebenenfallls anschliessendes Vermischen der gebildeten Chargetransfer-Komplexe mit Jod.

Die Metallkomplexe der Formel III sind bekannt [Helvetia Chimica Acta, Vol. 64, Fasc. 8, p 2544-2554 (1981)] oder können nach analogen Verfahren hergestellt werden. Auch Chargetransfer-Komplexe mit bis zu 4 Mol molekularem Jod sind dort beschrieben.

Eine Auswahl verschiednener Dibenzoletraaza-[14]-annulene wurden wie in Lin et al., J.C.S. Chem. Comm., 1980, 954ff beschrieben mit Iod oxidiert. Chargetransfer-Komplexe mit bis zu 7 Mol molekularem Iod sind beschrieben.

Zweckmässig wird bei der Oxidation unter Schutzgasatmosphäre und Feuchtigkeitsausschluß gearbeitet. Für die Oxidation mit Jod sind verschiedene Ausführungsformen möglich. Man kann Joddampf bei Raumtemperatur auf einen festen Metallkomplex der Formel III einwirken lassen, bis keine Jodaufnahme mehr erfolgt, oder man kann bei erhöhter Temperatur in einem Kosublimationsprozess die beiden Komponenten im gasförmigen Zustand miteinander reagieren lassen. Vorteilhafter ist es, die Oxidation in geeigneten inerten Lösungmitteln durchzuführen, bevorzugt in polaren, aprotischen, organischen Lösungsmitteln. Bei diesen Verfahren erhält man im allgemeinen Chargetransfer-Komplexe mit definierter Stöchiometrie in kristalliner bis pulveriger Form, die ein Molverhältnis von Metallkomplex zu molekularem Jod bis zu etwa 1 : 8 aufweisen können.

Zur Erzielung höherer Jodgehalte können die so erhaltenen Chargetransfer-Komplexe im gewünschten Verhältnis mit Jod vermischt werden, z. B. in üblichen Mischern mit schnellen Rühreinrichtungen, bei hohen Jodgehalten vorteilhaft in der Schmelze. Bei trockenen Pulvern ist auch eine Herstellung durch intensives Mahlen möglich.

Einfacher ist es, die Metallkomplexe der Formel III direkt im gewünschten Verhältnis mit Jod zu vermischen, was zweckmässig unter Schutzgasatmosphäre und Feuchtigkeitsausschluß erfolgt. Bis zu einem Jodgehalt von etwa 20 Mol molekularem Jod pro Mol Metallkomplex der Formel III ist ein Vermischen durch z. B. intensives Mahlen möglich, da feste pulverförmige Produkte gebildet werden. Bei höheren Jodgehalten wird das Festkörperverhalten zunehmend durch das weiche plastische Jod bestimmt und das Vermischen wird vorteilhafter in der Schmelze vorgenommen. Die Reaktionszeiten zur Bildung der Chargetransfer-Komplexe liegen im Bereich von Minuten bis zu wenigen Stunden, was sehr wirtschaftlich ist.

Bei den Chargetransfer-Komplexen bzw. deren Mischungen mit Jod entsprechend den Formeln I und II handelt es sich um Schwarze Festkörper, die eine elektrische Leitfähigkeit im Bereich von $10^{-1}$ bis etwa $10^{-4}$ S $cm^{-1}$ aufweisen. Sie sind gut verarbeitbar und eignen sich hervorragend als Kathodenmaterial in Lithium/Jod oder Silber/Jod Festelektrolytzellen (Batterien).

Die Batterien können in verschiedenen Formen ausgebildet sein, zum Beispiel als Knopfzellen, als flache Folienbatterien, zylindrische Batterien oder in Form von grösseren Batterien, die auch mehrere Zellen enthalten können. Die Batterien bestehen aus einem Becher aus elektrisch leitendem Material, das gegenüber dem Kathodenmaterial inert ist. Es kann sich hierbei um Metalle oder Metallegierungen handeln, wie z. B. rostfreier Stahl oder Chromnickelstahl. Der Deckel der Batterie besteht ebenfalls aus solchen Materialien. Zwischen Deckel und Becher befinden sich die Anode aus Lithium oder Silber und die Kathode aus den Komplexen der Formel I. Die Festelektrolytschicht aus LiJ bzw. AgJ bildet sich von selbst als Zwischenschicht durch den Kontakt von Anode und Kathode aus. Deckel und Becher werden durch eine geeignete Dichtungsmasse, z. B. aus Kunststoffen wie Polypropylen, abgedichtet.

Figur 1 zeigt die Entladungskurven von 3 Knopfzellen bei unterschiedlicher Stromabnahme; (dbtaa) steht dabei für Dibenzotetraazaannulen.

Die Herstellung der Batterien erfolgt in bekannter Weise durch Zusammenfügen der Einzelbestandteile unter Anwendung von Druck unter Schutzgasatmosphäre und Feuchtigkeitsausschluss.

Die Herstellung der Kathoden kann in bekannter Weise z. B. dergestalt erfolgen, dass man die mit Jod dotierten Komplexe oder deren Gemische mit Jod entsprechend der Formel I zu zum Beispiel Tabletten oder anderen Formen verpresst. Für dieses Verfahren sind trockene Pulver besonders geeignet. Bevorzugt verwendet man daher Chargetransfer-Komplexe mit einem Jodgehalt von 2 bis 50, besonders 2 bis 20 Mol molekularem Jod pro Mol Metallkomplex der, Formel III. In die Kathode können leitfähige Materialien

miteingearbeitet werden, die den elektrischen Kontakt zwischen Becher und Kathode verbessern, z. B. Drahtnetze aus Metallen, die noch mit dem Becher punktverschweisst werden können. Die Chargetransfer-Komplexe der Formel I und deren Gemische mit Jod können auch als Giessmasse zur Herstellung verschiedener Kathodenformen eingesetzt werden.

Die erfindungsgemäßen Batterien zeichnen sich durch eine relativ lange Lebensdauer aus. Eine mögliche Zerstörung durch Jod, beobachtbar an einem Jodaustritt, wird über einen längeren Zeitraum nicht gefunden Die erzielbare Spannung (Leerlaufspannung, OCV) beträgt 2,8 V und die inneren Widerstände für Knopfzellen der Grösse 2016 liegen im nichtentladenen Zustand etwa zwischen 0,2 und 1,2 Kiloohm.

Die erfindungsgemässen Batterien können zum Antrieb elektrischer Gerate mit kleinem Strombedarf verwendet werden. In Form von Knopfbatterien können sie beispielsweise als memory back up, in elektronischen Uhren oder Elektronenrechnern eingesetzt werden.

Die nachfolgenden Beispiele erläutern die Erfindung näher.

**A) Herstellungsbeispiele 1-15:**

Die in der folgenden Tabelle I beschriebenen Charge-Transferkomplexe werden nach folgenden Methoden hergestellt

**a) Herstellung durch Mischen in Jodschmelze**

1,7 g Metallkomplex der Formel III und Jod entsprechend den in Tabelle 1 angegebenen Molverhältnissen werden unter Feuchtigkeitsausschluss und unter Argon in einem 0,3 l Glaseinsatz eines Autoklaven gegeben und verschlossen. Darauf wird eine Stunde gerührt und danach die Schmelze abgekühlt.

**b) Herstellung durch Mischen der pulverförmigen Komponenten**

5 g Metallkomplex der Formel III und Jod entsprechend den Molverhältnissen in Tabelle 1 werden unter Wasserausschluß in einem Labormischer 2 Minuten bei voller Tourenzahl gemischt. Die Leitfähigkeiten nach 3 Tage Lagerung sind in Tabelle 1 angegeben.

**Tabelle 1**

| Beispiel No. | Herstell-methode | Metallkomplex der Formel III (A) M | $R^1$ | $R^2$ | $R^3$ | $R^4$ | $R^5$ | $R^6$ | Molver-verhältnis A : $J_2$ | Leitfähigkeit bei 25°C (S $cm^{-1}$) |
|---|---|---|---|---|---|---|---|---|---|---|
| 1 | b | Ni | H | H | H | H | H | H | 1 : 10 | $1 \cdot 10^{-2}$ |
| 2 | b | Ni | H | H | H | H | H | H | 1 : 20 | $3 \cdot 10^{-3}$ |
| 3 | a | Ni | H | H | H | H | H | H | 1 : 53,2 | $2,5 \cdot 10^{-3}$ |
| 4 | b | Ni | $CH_3$ | $CH_3$ | H | H | H | H | 1 : 10 | $1 \cdot 10^{-3}$ |
| 5 | a | Ni | $CH_3$ | $CH_3$ | H | H | H | H | 1 : 65,6 | $9,4 \cdot 10^{-4}$ |
| 6 | a | Ni | H | H | $CH_3$ | H | $CH_3$ | H | 1 : 53,2 | $1,7 \cdot 10^{-4}$ |
| 7 | a | Ni | $CH_3$ | $CH_3$ | $CH_3$ | H | $CH_3$ | H | 1 : 75,5 | $9,6 \cdot 10^{-4}$ |
| 8 | a | Ni | $CH_3$ | $CH_3$ | $CH_3$ | $CH_3$ | $CH_3$ | $CH_3$ | 1 : 113,3 | $1,6 \cdot 10^{-3}$ |
| 9 | a | Ni | p-Toluyl | | H | H | H | H | 1 : 88,3 | $7 \cdot 10^{-4}$ |
| 10 | a | Cu | H | H | H | H | H | H | 1 : 21,8 | $3,3 \cdot 10^{-3}$ |
| 11 | b | Fe | $CH_3$ | $CH_3$ | H | H | H | H | 1: 20,4 | $4,5 \cdot 10^{-2}$ |
| 12 | a | Ni | H | H | -CH=CH-C=CH- | | -CH=CH-CH=CH- | | 1 : 44,8 | $4 \cdot 10^{-4}$ |
| 13 | a | Ni | H | H | H | H | H | H | 1 : 200 | $2 \cdot 10^{-5}$ |
| 14 | b | Ni | H | H | $-OCH_2CH_2O-$ | | $-OCH_2CH_2O-$ | | 1 : 20 | $2 \cdot 10^{-4}$ |
| 15 | b | Ni | $CH_3$ | $CH_3$ | $-OCH_2O-$ | | $-OCH_2O-$ | | 1 : 20 | $1 \cdot 10^{-4}$ |

**B) Anwendungsbeispiele 16-25:**

Die in nachfolgender Tabelle 2 aufgeführten Komplexe der Formel I bzw. Mischungen mit Jod werden nach Methode a (Beispiele 16 und 17) und nach Methode b (Beispiele 14, 15 und 18-23) hergestellt und unter einem Druck von 147 MPa zu Pellets von 15,9 mm Durchmesser und 0,8 mm Dicke verpresst. Unter dem gleichen Druck wird anschliessend ein Netz aus rostfreiem Stahl eingepresst. Danach werden Knopfzellen von 1,6 mm Dicke und 20 mm Durchmesser gemäss dem Aufbau der beiliegenden Figur 1 hergestellt.

Kathodenbecher und Anodendeckel bestehen je aus 0,2 mm dickem rostfreiem Stahl. Die Lithiumanode wird aus einer 0,45 mm dicken Folie ausgestanzt. Die Knopfzelle wird mit Hilfe einer Dichtung aus Polypropylen abgedichtet. Die Herstellung der Knopfzelle erfolgt unter Argon als Schutzgas.

In der Tabelle 2 sind neben der Zusammensetzung des Kathodenmaterials die Spannung und der innere Widerstand $R_i$ der Zelle im nichtentladenen Zustand angegeben.

**Tabelle 2**

| Beispiel | Metallkomplex der Formel I | | | | | | | | OCV | $R_i$ |
|---|---|---|---|---|---|---|---|---|---|---|
| No. | M | $R^1$ | $R^2$ | $R^3$ | $R^4$ | $R^5$ | $R^6$ | x | (Volt) | (kΩ) |
| 16 | Ni | H | H | H | H | H | H | 10 | 2,806 | 0,434 |
| 17 | Ni | H | H | H | H | H | H | 10 | 2,804 | 0,587 |
| 18 | Ni | H | H | H | H | H | H | 50 | 2,806 | 0,726 |
| 19 | Ni | H | H | H | H | H | H | 50 | 2,802 | 1,02 |
| 20 | Ni | $CH_3$ | $CH_3$ | H | H | H | H | 5 | 2,804 | 0,691 |
| 21 | Ni | $CH_3$ | $CH_3$ | H | H | H | H | 5 | 2,803 | 0,725 |
| 22 | Ni | $CH_3$ | $CH_3$ | H | H | H | H | 10 | 2,806 | 0,902 |
| 23 | Ni | $CH_3$ | $CH_3$ | H | H | H | H | 10 | 2,805 | 0,732 |
| 24 | Ni | $CH_3$ | $CH_3$ | H | H | H | H | 20 | 2,807 | 0,351 |
| 25 | Ni | $CH_3$ | $CH_3$ | H | H | H | H | 20 | 2,809 | 0,247 |

**Patentansprüche**

1. Festelektrolytzelle enthaltend eine feste Anode aus Lithium oder Silber, einen festen Lithiumjodid oder Silberjodid-Elektrolyt und einen Chargetransfer-Komplex aus einem Metallkomplex und Jod als Kathode, dadurch gekennzeichnet, dass die Kathode aus einem mit Jod dotierten Komplex oder dessen Gemisch mit Jod entsprechend der Formel I besteht,

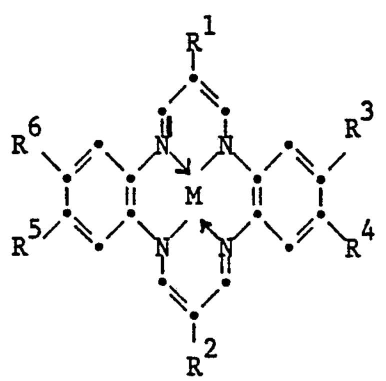

• x $J_2$ (I)

worin

M ein zweiwertiges Metallatom aus der Gruppe Fe, Co, Ni, Cu, Zn, Pd und Pt ist,

$R^1$ und $R^2$ unabhängig voneinander für ein Wasserstoffatom, oder unsubstituiertes oder substituiertes Alkyl, Cycloalkyl, Aryl, Aralkyl, Alkaralkyl oder Acyl stehen, $R^3$, $R^4$, $R^5$ und $R^6$ unabhängig voneinander ein Wasserstoffatom, Alkyl, Alkoxy, Alkylthio und Alkoxycarbonyl mit 1 bis 8 C-Atomen, Aryloxy oder Halogen sind, oder $R^3$ und $R^4$ sowie $R^5$ und $R^6$ zusammen $-(CH_2)_n-$ mit n = 3, 4 oder 5, $-O(CH_2)_mO-$ mit m = 1 bis 4 oder ein Rest der Formel -CH = CH-CH = CH- sind, und x für eine rationale Zahl von 1 bis 300 steht.

2. Festelektrolytzelle gemäss Anspruch 1, dadurch gekennzeichnet, dass M in Formel 1 für Fe und besonders für Cu oder Ni steht.

3. Festelektrolytzelle gemäss Anspruch 1, dadurch gekennzeichnet, dass $R^1$ und $R^2$ in Formel I als Alkyl 1 bis 6 C-Atome, als Cycloalkyl 5 oder 6 Ringkohlenstoffatome und als Acyl 1 bis 6 C-Atome enthalten, als Aryl Phenyl, als Alkaryl Alkylphenyl und als Alkaralkyl Alkylbenzyl sind.

4. Festelektrolytzelle gemäss Anspruch 1, dadurch gekennzeichnet, dass $R^1$ und $R^2$ ein Wasserstoffatom, Methyl, Äthyl, Phenyl, p-Methylphenyl, p-Methoxyphenyl, p-Chlorphenyl oder p-Fluorphenyl und $R^3$, $R^4$, $R^5$ und $R^6$ ein Wasserstoffatom, Methyl, Äthyl, Methoxy, Methylthio oder Fluor oder $R^3$ und $R^4$ sowie $R^5$, $R^6$ zusammen $-(CH_2)_4-$, $-OCH_2O-$, $-OCH_2CH_2O-$ oder $-CH=CH-CH=CH-$ sind.

5. Festelektrolytzelle gemäss Anspruch 1, dadurch gekennzeichnet, dass x in Formel I eine Zahl von 1 bis 200, besonders 1 bis 100 ist.

6. Festelektrolytzelle gemäss Anspruch 5, dadurch gekennzeichnet, dass x in Formel I eine Zahl von 2 bis 50 ist.

7. Festelektrolytzelle gemäss Anspruch 1, dadurch gekennzeichnet, dass $R^1$ bis $R^6$ in Formel I für ein Wasserstoffatom oder Methyl und M für Cu oder Ni stehen.

8. Mit Jod dotierter Komplex oder dessen Gemische mit Jod entsprechend der Formel II

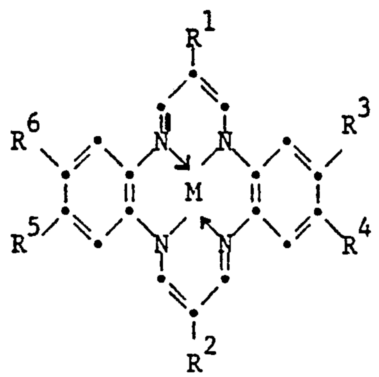 • y $J_2$ (II),

worin M ein zweiwertiges Metallatom aus der Gruppe Fe, Co, Ni, Cu, Zn, Pd und Pt ist, $R^1$ und $R^2$ unabhängig voneinander für ein Wasserstoffatom, oder unsubstituiertes oder Substituiertes Alkyl, Cycloalkyl, Aryl, Aralkyl, Alkaralkyl oder Acyl stehen, $R^3$, $R^4$, $R^5$ und $R^6$ unabhängig voneinander ein Wasserstoffatom, Alkyl, Alkoxy, Alkylthio und Alkoxycarbonyl mit 1 bis 8 C-Atomen, Aryloxy oder Halogen sind, oder $R^3$ und $R^4$ sowie $R^5$ und $R^6$ zusammen $-(CH_2)_n-$ mit n = 3, 4 oder 5, $-O(CH_2)_mO-$ mit m 1 bis 4 oder ein Rest der Formel $CH=CH-CH=CH-$ sind, und y für eine rationale Zahl von 10 bis 300 steht.

9. Verfahren zur Herstellung von Komplexen oder deren Gemischen mit Jod entsprechend der Formel II gemäss Anspruch 8, dadurch gekennzeichnet, dass man einen Metallkomplex der Formel III

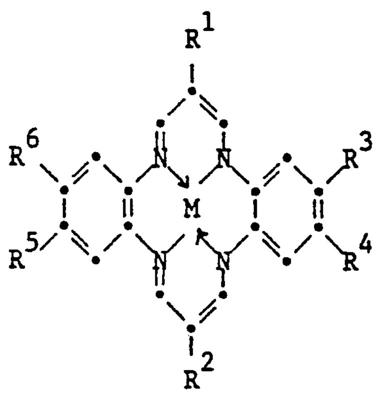 (III),

worin M und $R^1$ bis $R^6$ die in Anspruch 8 angegebene Bedeutung haben, mit y Mol Jod oxidiert, wobei y eine rationale Zahl von 10 bis 300 ist.

10. Verwendung von mit Jod dotierten Komplexen oder deren Gemischen mit Jod entsprechend der Formel I gemäss Anspruch 1 als Kathodenmaterial in Jod/Lithium-Batterien oder Jod/Silber-Batterien.

**Claims**

1. A solid electrolyte cell containing a solid anode made of lithium or silver, a solid lithium iodide or silver iodide electrolyte and a charge-transfer complex consisting of a metal complex, and iodine as the cathode, wherein the cathode consists of an iodine-doped complex or a mixture thereof with iodine having the formula I

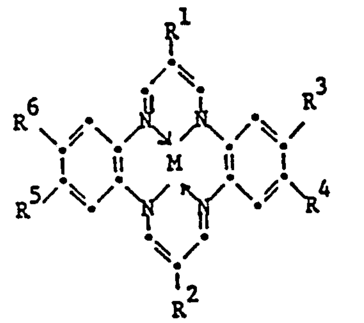

$\cdot x J_2$ (I)

in which M is a divalent metal atom belonging to the group consisting of Fe, Co, Hi, Cu, Zn, Pd and Pt, $R^1$ and $R^2$ independently of one another are a hydrogen atom or alkyl, cycloalkyl, aryl, aralkyl, alkaralkyl or acyl each of wich is unsubstituted or substituted, $R^3$, $R^4$, $R^5$ and $R^6$ independently of one another are a hydrogen atom, alkyl, alkoxy, alkylthio and alkoxycarbonyl having 1 to 8 C atoms, aryloxy or halogen, or $R^3$ and $R^4$ and also $R^5$ and $R^6$ together are $-(CH_2)_n-$ in which n = 3, 4 or 5, $-O-(CH_2)_mO-$ in which m = 1 to 4 a radical of the formula $-CH=CH-CH=CH-$, and x is a rational number from 1 to 300.

2. A solid electrolyte cell according to Claim 1, wherein M in formula I is Fe and especially Cu or Ni.

3. A solid electrolyte cell according to Claim 1, wherein $R^1$ and $R^2$ in formula I contain 1 to 6 C atoms as alkyl, 5 or 6 ring carbon atoms as cycloalkyl and 1 to 6 C atoms as acyl, and, as aryl are phenyl, as alkaryl are alkylphenyl and as alkaralkyl are alkylbenzyl.

4. A solid electrolyte cell according to Claim 1, wherein $R^1$ and $R^2$ are a hydrogen atom, methyl, ethyl, phenyl, p-methylphenyl, p-methoxyphenyl, p-chlorophenyl or p-fluorophenyl and $R^3$, $R^4$, $R^5$ and $R^6$ are a hydrogen atom, methyl, ethyl, methoxy, methylthio or fluorine or $R^3$ and $R^4$ and also $R^5$ and $R^6$ together are $-(CH_2)_4-$, $-OCH_2O-$, $-OCH_2CH_2O-$ or $-CH=CH-CH=CH-$.

5. A solid electrolyte cell according to Claim 1, wherein x in formula 1 is a number from 1 to 200, especially 1 to 100.

6. A solid electrolyte cell according to Claim 5, wherein x in formula I is a number from 2 to 50.

7. A solid electrolyte cell according to Claim 1, wherein $R^1$ to $R^6$ in formula I are a hydrogen atom or methyl and M is Cu or Ni.

8. An iodine doped complex or mixtures thereof with iodine having the formula II

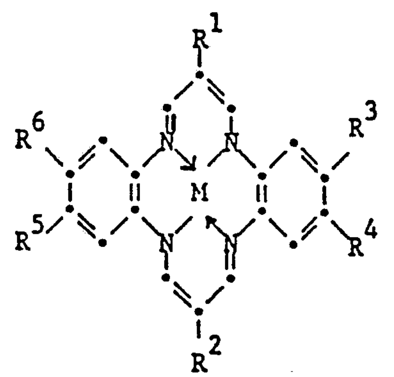

$\cdot y J_2$ (II)

in which M is a divalent metal atom belonging to the group consisting of Fe, Co, Hi, Cu, Zn, Pd and Pt, $R^1$ and $R^2$ independently of one another are a hydrogen atom or alkyl, cycloalkyl, aryl, aralkyl, alkaralkyl or acyl each of which is unsubstituted or substituted, $R^3$, $R^4$, $R^5$ and $R^6$ independently of one another are a hydrogen atom, alkyl, alkoxy, alkylthio and alkoxycarbonyl having 1 to 8 C atoms, aryloxy or halogen, or $R^3$ and $R^4$ and also $R^5$ and $R^6$ together are $-(CH_2)_n-$ in which n = 3, 4 or 5, $-O(CH_2)_mO-$ in which m = 1 to 4 or a radical of the formula $-CH=H-CH=CH-$, and y is a rational number from 10 to 300.

9. A process for the preparation of complexes or mixtures thereof with iodine having the formula II according to Claim 8, which comprises oxidising a metal complex of the formula III

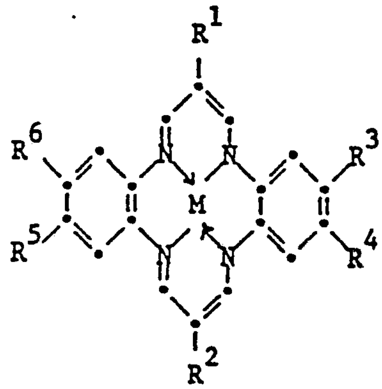

(III)

in which M and $R^1$ to $R^6$ have the meaning indicated in claim 1, by means of y moles of iodine, y being a rational number from 10 to 300.

10. The use of iodine-doped complexes or mixtures thereof with iodine having the formula 1 according to Claim 1 as the cathode material in iodine/lithium batteries or iodine/silver batteries.

**Revendications**

1. Elément de pile à électrolyte solide contenant une anode solide en lithium ou en argent, un électrolyte solide à l'iodure de lithium ou à l'iodure d'argent et un complexe de transfert de charges formé d'un complexe de métal et d'iode comme cathode, élément caractérisé en ce que la cathode consiste en un complexe dopé par de l'iode ou en son mélange avec l'iode, répondant à la formule I

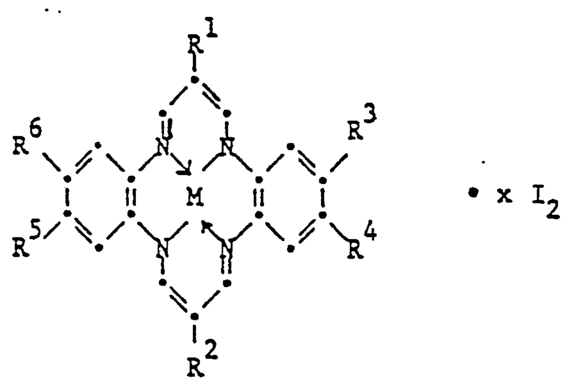

(I)

dans laquelle:

M représente un atome de métal divalent choisi parmi Fe, Co, Ni, Cu, Zn, Pd et Pt,

$R^1$ et $R^2$ représentent, indépendamment l'un de l'autre, un atome d'hydrogène ou un groupe alkyle, cycloalkyle, aryle, aralkyle, alkylaralkyle ou acyle, substitués ou non substitués; $R^3$, $R^4$, $R^5$ et $R^6$ représentent, indépendamment l'un de l'autre, un atome d'hydrogène, un groupe alkyle, alcoxy, alkylthio et alcoxycarbonyle ayant 1 à 8 atomes de carbone, aryloxy ou de l'halogène, ou bien $R^3$ et $R^4$ ainsi que $R^5$ et $R^6$ forment ensemble $-(CH_2)_n-$ avec n valant 3, 4 ou 5, $-O(CH_2)_m-$ avec m valant 1 à 4, ou un reste de formule -CH=CH-CH=CH, et x est un nombre rationnel valant 1 à 300.

2. Elément de pile à électrolyte solide selon la revendication 1, caractérisé en ce que, dans la formule I, M représente Fe et en particulier Cu ou Ni.

3. Elément de pile à électrolyte solide selon la revendication 1, caractérisé en ce que, dans la formule I, les groupes $R^1$ et $R^2$ contiennent, en tant que groupe alkyle, 1 à 6 atomes de carbone, en tant que groupe cycloalkyle 5 ou 6 atomes de carbone de noyau et tant que groupe acyle 1 à 6 atomes de carbone et, en tant qu'aryle, ils représentent un groupe phényle, en tant qu'alkaryle un groupe alkylphényle et en tant qu'alkylaralkyle un groupe alkylbenzyle.

4. Elément de pile à électrolyte solide selon la revendication 1, caractérisé en ce que $R^1$ et $R^2$ représentent un atome d'hydrogène, un groupe méthyle, éthyle, phényle, p-méthylphényle, p-méthoxyphényle, p-chlorophényle ou p-fluorophényle, et $R^3$, $R^4$, $R^5$ et $R^6$ representent un atome d'hydrogène, un groupe méthyle, éthyle, méthoxy, méthylthio ou du fluor, ou bien $R^3$ et $R^4$ ainsi que $R^5$ et $R^6$ forment ensemble $-(CH_2)_4-$, $-OCH_2O-$, $-OCH_2CH_2O-$ ou -CH=CH-CH=CH-.

5. Elément de pile à électrolyte solide selon la revendication 1, caractérisé en ce que, dans la formule I, x est un nombre valant 1 à 200, en particulier 1 à 100.

6. Elément de pile à électrolyte solide selon la revendication 5, caractérisé en ce que, dans la formule I, x est un nombre valant 2 à 50.

7. Elément de pile à électrolyte solide selon la revendication 1, caractérisé en ce que, dans la formule I, les symboles $R^1$ à $R^6$ représentent chacun un atome d'hydrogène ou un groupe méthyle et M représente Cu ou Ni.

8. Complexe dopé à l'iode ou ses mélanges avec l'iode, répondant à la formule II :

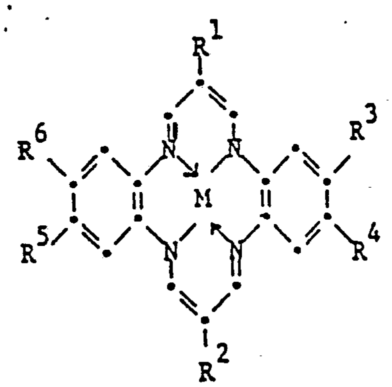

$\cdot\ y\ I_2$ (II),

dans laquelle M représente un atome de métal divalent choisi parmi Fe, Co, Ni, Cu, Zn, Pd et Pt,

$R^1$ et $R^2$ représentent, indépendamment l'un de l'autre, un atome d'hydrogène, ou un groupe alkyle, cycloalkyle, aryle, aralkyle, alkylaralkyle ou acyle, substitués ou non substitués; $R^3$, $R^4$, $R^5$ et $R^6$ representent, indépendamment l'un de l'autre, un atome d'hydrogène, ou un groupe alkyle, alcoxy, alkylthio et alcoxycarbonyle ayant 1 à 8 atomes de carbone, aryloxy ou un atome d'halogène, ou bien $R^3$ et $R^4$ ainsi que $R^5$ et $R^6$ forment ensemble $-(CH_2)_n-$ avec n valant 3, 4 ou 5, $-O(CH_2)_mO-$ avec m valant 1 à 4, ou un reste de formule $-CH=CH-CH=CH-$, et y est un nombre rationnel valant 10 à 300.

9. Procédé pour préparer des complexes, ou leurs mélanges avec l'iode, répondant à la formule II selon la revendication 8, procédé caractérisé en ce qu'on oxyde un complexe de métal de formule III :

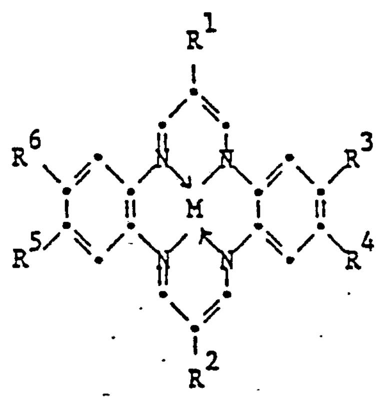

(III),

(dans laquelle M et $R^1$ à $R^6$ ont le sens indiqué dans la revendication 8) avec y moles d'iode, y désignant un nombre rationnel valant de 10 à 300.

10. Utilisation de complexes dopés à l'iode ou de leurs mélanges avec l'iode, répondant à la formule 1 selon la revendication 1, comme matière de cathode dans des piles à l'iode/lithium ou des piles à l'iode/argent.

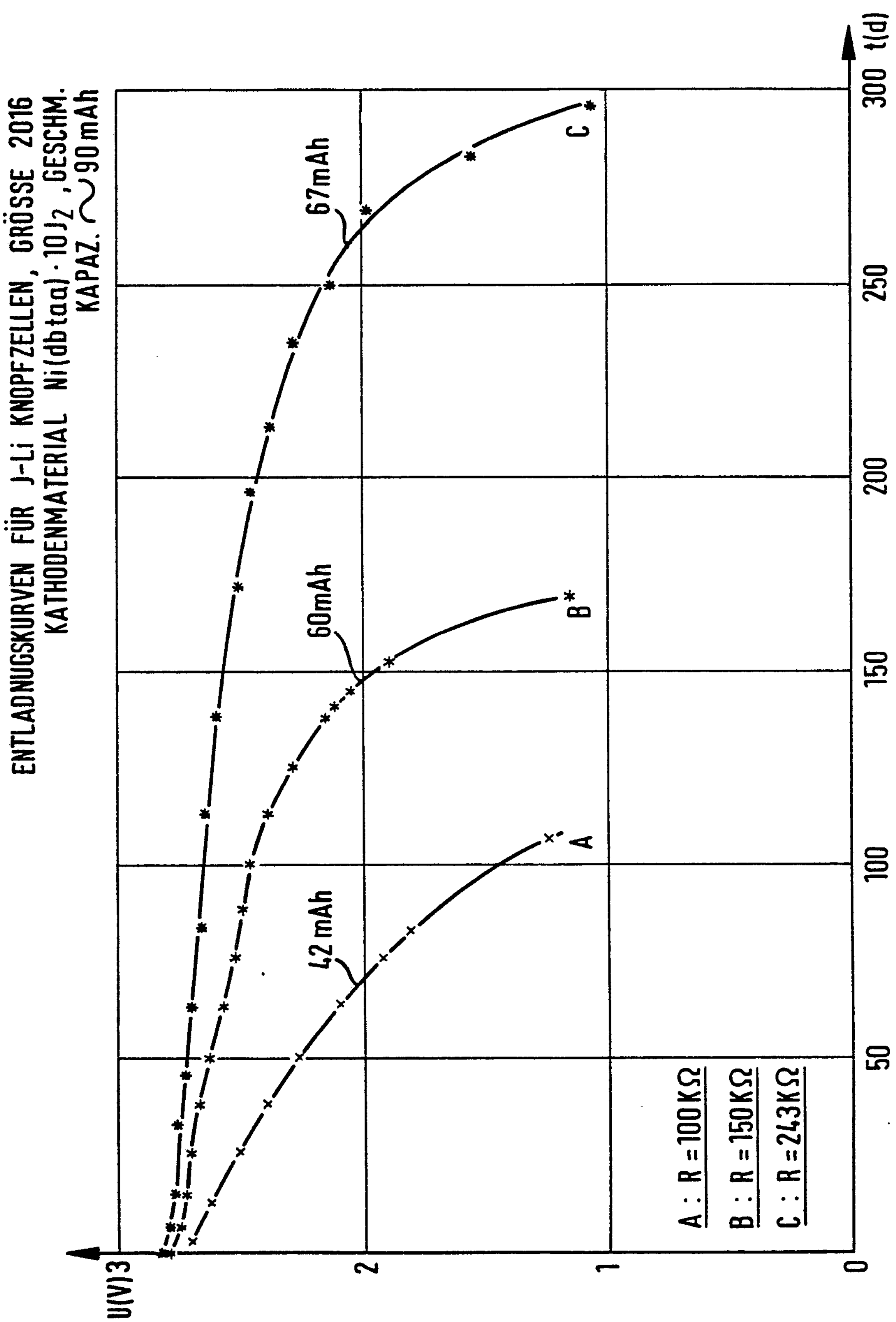
ENTLADNUGSKURVEN FÜR J-Li KNOPFZELLEN, GRÖSSE 2016
KATHODENMATERIAL Ni(dbtaa)·10J2 ,GESCHM.
KAPAZ. ~90 mAh
U(V)
3
2
1
0
50
100
150
200
250
300
t(d)
42 mAh
60mAh
67mAh
A
B*
C *
A : R = 100 KΩ
B : R = 150 KΩ
C : R = 243 KΩ

Fig. 1